(19) Europäisches Patentamt
European Patent Office
Office européen des brevets

(11) Veröffentlichungsnummer: **0 358 097**
**A1**

(12) # EUROPÄISCHE PATENTANMELDUNG

(21) Anmeldenummer: **89115964.2**

(22) Anmeldetag: **30.08.89**

(51) Int. Cl.5: **C07C 309/07 , C07C 303/02 , C11D 1/12**

(30) Priorität: **08.09.88 DE 3830569**

(43) Veröffentlichungstag der Anmeldung: **14.03.90 Patentblatt 90/11**

(84) Benannte Vertragsstaaten: **ES**

(71) Anmelder: **Henkel Kommanditgesellschaft auf Aktien**
**Postfach 1100 Henkelstrasse 67**
**D-4000 Düsseldorf 1(DE)**

(72) Erfinder: **Fabry, Bernd, Dr.**
**Danziger Strasse 31**
**D-4052 Korschenbroich(DE)**
Erfinder: **Westfechtel, Alfred, Dr.**
**Kerschensteinerweg 9**
**D-4010 Hilden(DE)**
Erfinder: **Eierdanz, Horst, Dr.**
**Am Eichelkamp 116**
**D-4010 Hilden(DE)**

(54) **Sulfonierte Fettketone, Verfahren zu ihrer Herstellung und ihre Verwendung als oberflächenaktive Mittel.**

(57) Sulfonierte Fettketone, erhältlich durch Sulfonierung von Fettketonen der allgemeinen Formel I

$R^1$-CO-$R^2$ (I)

in der

$R^1$ einen Kohlenwasserstoffrest mit 15 bis 21 Kohlenstoffatomen und einer olefinischen Doppelbindung oder mehreren und

$R^2$ einen gegebenenfalls eine olefinische Doppelbindung oder mehrere aufweisenden Kohlenwasserstoffrest mit 1 bis 21 Kohlenstoffatomen

bedeuten,

sowie anschließender Neutralisation und Hydrolyse bzw. Alkoholyse der Sulfonierungsprodukte, sind wasserlösliche Verbindungen mit interessanten oberflächenaktiven Eigenschaften.

EP 0 358 097 A1

## Sulfonierte Fettketone, Verfahren zu ihrer Herstellung und ihre Verwendung als oberflächenaktive Mittel.

Die Erfindung betrifft sulfonierte Fettketone, erhältlich durch Sulfonierung von Fettketonen der allgemeinen Formel I

$R^1$-CO-$R^2$ (I)

in der

$R^1$ einen Kohlenwasserstoffrest mit 15 bis 21 Kohlenstoffatomen und einer olefinischen Doppelbindung oder mehreren und

$R^2$ einen gegebenenfalls eine olefinische Doppelbindung oder mehrere aufweisenden Kohlenwasserstoffrest mit 1 bis 21 Kohlenstoffatomen

bedeuten, sowie anschließender Neutralisation und Hydrolyse bzw. Alkoholyse der Sulfonierungsprodukte.

Es ist bekannt, daß langkettige Dialkyl- und/oder -alkenyl-Ketone (Fettketone) unter anderem als Antischaummittel eingesetzt werden können, vgl. DE-A 25 53 990. Ein wesentlicher Nachteil dieser Fettketone besteht in ihrer schlechten Wasserlöslichkeit.

Die vorliegende Erfindung beruht auf der Erkenntnis, daß sich ungesättigte Fettketone der obigen Formel I durch Sulfonierung in gut wasserlösliche Verbindungen mit interessanten oberflächenaktiven Eigenschaften überführen lassen.

Die als Ausgangsverbindungen einzusetzenden Fettketone der allgemeinen Formel I können durch übliche Pyrolyse von Erdalkalisalzen höherer Fettsäuren, gegebenenfalls im Gemisch mit Erdalkalisalzen niederer Fettsäuren, sowie gegebenenfalls in Anwesenheit von geeigneten Katalysatoren erhalten werden, z.B. gemäß den in der DE-A 25 53 990 sowie der DE-B 27 58 113 sowie in Organic Synthesis, Vol. 33, S. 854 ff., beschriebenen Verfahren.

Geeignete Fettketone für die Herstellung der Verbindungen der Erfindung sind insbesondere solche, die bei der Pyrolyse von Erdalkalisalzen von Fettsäuren mit 16 bis 22 Kohlenstoffatomen und einer olefinischen Doppelbindung oder mehreren erhalten werden, wobei Fettsäuren der genannten Kettenlänge mit einer einzigen olefinischen Doppelbindung bevorzugt sind. Derartige Fettsäuren können synthetischen und/oder natürlichen Ursprungs sein. Natürliche Fettsäuren der hier angesprochenen Art sind insbesondere Öl-, Elaidin-, Petroselin-, Chaulmoogra-, Gadolein- und Erucasäure. Da diese Fettsäuren, wie in der Fettchemie üblich, aus natürlichen, insbesondere tierischen oder pflanzlichen Fetten und Ölen hergestellt werden, z.B. aus Sonnenblumenöl, Koreanderöl, Chaulmoograöl, Rapsöl, Rüböl, Rindertalg, Schweineschmalz und dergleichen, werden sie zumeist in Form ihrer an der jeweiligen Fettsäure reichen technischen Gemische eingesetzt, die noch andere, höhere und/oder niedrigere Fettsäuren enthalten könne. Für die sulfonierten Fettketone der Erfindung können im übrigen auch Fettketone eingesetzt werden, die durch Pyrolyse von Gemischen von Erdalkalisalzen von lang- und kurzkettigen Fettsäuren entstehen und neben einer ungesättigten langen Kohlenwasserstoffkette mit 15 bis 21 Kohlenstoffatomen und insbesondere einer olefinischen Doppelbindung auch eine kurzkettige, gesättigte Kohlenwasserstoffkette mit 1 bis 7 Kohlenstoffatomen, eine gesättigte Kohlenwasserstoffkette mit 8 bis 15 Kohlenstoffatomen oder eine gesättigte oder ungesättigte Kohlenwasserstoffkette mit 10 bis 21 Kohlenstoffatomen aufweisen können. Während somit die Gruppe $R^1$ der Fettketone der allgemeinen Formel I immer von Fettsäuren mit 16 bis 22 Kohlenstoffatomen und mindestens einer olefinischen Doppelbindung oder mehreren abgeleitet ist, kann der Rest $R^2$ von beliebigen, gesättigten aliphatischen Carbonsäuren oder ungesättigten aliphatischen Carbonsäuren mit mindestens einer olefinischen Doppelbindung mit 2 bis 22 Kohlenstoffatomen abgeleitet sein, z.B. von Essigsäure, Propionsäure, Buttersäure, Pentan-, Hexan, Heptan-, Octan-, Nonan-, Decan-, Undecan-, Undecylen-, Dodecan-, Tetradecan-, Hexadecan-, Hexadecen-, Octadecan-, Octadecen-, Eicosan-, Eicosen-, Docosan- und Docosensäure. Bevorzugte Fettketone der allgemeinen Formel I für die Herstellung der sulfonierten Fettketone der Erfindung sind dabei diejenigen, in denen die Gruppen $R^1$ und $R^2$ identisch sind, d.h. jeweils 15 bis 21 Kohlenstoffatome und eine olefinische Doppelbindung oder mehrere, insbesondere eine olefinische Doppelbindung aufweisen. Besonders bevorzugte Reste $R^1$ und $R^2$ sind diejenigen aus der von Pentadecylen, Heptadecylen, 1-Dodecyl-cyclopenten-2, Nonadecylen und Heneicosylen gebildeten Gruppe.

Die Sulfonierung der Fettketone der allgemeinen Formel I kann mit für die Sulfonierung von Olefinen üblichen Sulfonierungsmitteln erfolgen, z.B. mit Schwefelsäure, Chlorsulfonsäure, Oleum, Amidosulfonsäure oder Schwefeltrioxid, wobei insbesondere gasförmiges Schwefeltrioxid bevorzugt ist. Die Sulfonierung kann in der für Fettsäureniedrigalkylester bekannten Weise erfolgen, z.B. mit gasförmigem Schwefeltrioxid in hierfür geeigneten Reaktoren, insbesondere vom Typ der Fallfilmreaktoren. Dabei wird das Schwefeltrioxid mit Luft oder Stickstoff verdünnt und vorzugsweise in Form eines Gasgemisches mit ca. 1 bis 8, insbesondere 3 bis 5 Vol.-% Schwefeltrioxid eingesetzt. Bevorzugt wird die Reaktion in Abwesenheit von

Lösemitteln durchgeführt; est können jedoch auch sämtliche für die Sulfonierung ungesättigter Fettsäureester, Olefine, Aromaten und dergleichen üblichen Lösemittel eingesetzt werden.

Gemäß einer weiteren vorteilhaften Ausführungsform der Erfindung erfolgt die Sulfonierung in einem Verhältnis von 0,5 bis 1,8, insbesondere 0,6 bis 1,5 und vorzugsweise 1,0 bis 1,3 Mol Schwefeltrioxid pro Mol der in den Fettketonen der allgemeinen Formel I enthaltenen olefinischen Doppelbindungen. Eine bevorzugte Sulfonierungstemperatur liegt im Bereich von 15 bis 80, insbesondere von 40 bis 70 °C.

Wenn die erfindungsgemäß eingesetzten Fettketone der Formel I in der Gruppe $R^1$ und gegebenenfalls auch in der Gruppe $R^2$ mindestens je eine olefinische Doppelbindung aufweisen, können sie bei der Herstellung der Titelverbindungen mit mindestens 2 mol Schwefeltrioxid (je 1 mol pro Doppelbindung) reagieren. Es ist jedoch nicht erforderlich, sämtliche olefinischen Doppelbindungen der eingesetzten Fettketone zu sulfonieren, so daß man z.B. auch mit durchschnittlich 1 mol Schwefeltrioxid sulfonierte Fettketone aus Fettketonen mit ungesättigten Fettalkylresten $R^1$ und $R^2$ erhalten kann.

Die erfindungsgemäß erhaltenen Sulfonierungsprodukte werden mit Basen, insbesondere wässrigen und/oder alkoholischen Basen, vorzugsweise unter Einhaltung eines pH-Wertes im Bereich von 6 bis 14, insbesondere von 8 bis 12, neutralisiert. Als Basen kommen hierbei Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak und organische Basen, bevorzugt Amine, besonders bevorzugt Mono-, Di- bzw. Tri-$C_1$-$C_4$-alkylamine und Mono-, Di- bzw. Tri- $C_2$-$C_4$-hydroxyalkylamine, in Betracht. Alkalimetall- und Erdalkalimetalloxide und Ammoniak kommen dabei vorzugsweise in Form mehr oder weniger konzentrierter wässriger und/oder alkoholischer Lösungen zum Einsatz. Für die Neutralisation mit wasserfreien Basen eignen sich auch Alkali- und Erdalkalialkoxide.

Bei der Herstellung der sulfonierten Fettketone der Erfindung durch Sulfonierung von Fettketonen der allgemeinen Formel I können sich intermediär cyclische bzw. verbrückte Sulfonierungsprodukte bilden, die einer Hydrolyse bzw. Alkoholyse zugeführt werden müssen, um ein allmähliches Nachsäuern der Produkte zu vermeiden. Diese Hydrolyse bzw. Alkoholyse erfolgt zweckmäßigerweise zusammen mit der Neutralisation, wobei Reaktionstemperaturen von mindestens 70 °C und Reaktionszeiten von 15 bis 240 min bevorzugt sind. Die Obergrenze der Hydrolysetemperatur ist durch den Siedepunkt des Wassers bestimmt, wobei man jedoch auch bei Temperaturen über 100 °C unter Druck hydrolysieren kann.

Wenn die eingesetzten Fettketone, wie bei der Herstellung aus technischen Fettsäureschnitten durchaus möglich ist, Anteile an gesättigten Fettketonen enthalten, können diese in den sulfonierten Fettketonen verbleiben oder, falls erwünscht, in an sich bekannter Weise, z.B. durch Phasentrennung, abgetrennt werden. Entsprechende Verfahren sind an sich für Sulfonate von Niedrigalkylestern ungesättigter Fettsäuren bekannt, vgl. EP-A 0 130 759.

Die Erfindung ist weiterhin auf Verfahren zur Herstellung von sulfonierten Fettketonen mit den oben genannten Merkmalen sowie auf die Verwendung der sulfonierten Fettketone der Erfindung als oberflächenaktive Mittel gerichtet.

Weiterhin betrifft die Erfindung oberflächenaktive Mittel, die einzeln oder in Mischung sulfonierte Fettketone der allgemeinen Formel II

$R^3$-CO-$R^4$ (II)

enthalten,

in der

$R^3$ eine Gruppe der Formel IIIa bzw. IIIb

$CH_3$-$(CH_2)_x$-CH($OR^5$)-$(CH_2)_y$-CH($SO_3M$)-$(CH_2)_z$-$CH_2$- (IIIa)

$CH_3$-$(CH_2)_x$-CH($SO_3M$)-$(CH_2)_y$-CH($OR^5$)-$(CH_2)_z$-$CH_2$- (IIIb)

in denen

$R^5$ Wasserstoff oder Alkyl mit 1 bis 3 Kohlenstoffatomen,

x und z Zahlen im Bereich von 0 bis 17,

y die Zahlen 0,1 oder 2 und

M ein Alkalimetall- oder ein Äquivalent eines Erdalkalimetallions oder ein gegebenenfalls mono-, di- oder tri-$C_1$-$C_4$-alkyl- bzw. mono-, di- oder tri-$C_2$-$C_4$-hydroxyalkylsubstituiertes Ammoniumion ist,

wobei die Summe von x + y + z eine Zahl im Bereich von 13 bis 17 ergibt,

oder $R^3$ einen Rest, der durch Abspaltung eines Moleküls Wasser aus den Gruppen IIIa bzw. IIIb gebildet ist,

und

$R^4$ wie $R^3$ definiert ist oder einen gegebenenfalls eine olefinische Doppelbindung oder mehrere aufweisenden Kohlenwasserstoffrest mit 1 bis 21 Kohlenstoffatomen

bedeutet.

Weiterhin betrifft die Erfindung oberflächenaktive Mittel, die sulfonierte Fettketone der allgemeinen

Formel IV

$R^6$-$(CH_2)_{13}$-CO-$(CH_2)_{13}$-$R^7$ (IV)

enthalten,

in der

$R^6$ eine Gruppe der Formel V

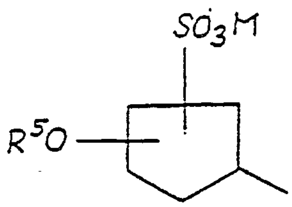

(V)

wobei $R^5$ und M wie oben definiert sind,

oder $R^6$ einen Rest bedeutet, der durch Abspaltung eines Moleküls Wasser aus der Gruppe der Formel V gebildet ist,

und

$R^7$ einen Cyclopent-(2)-yl-Rest bedeutet oder wie $R^6$ definiert ist.

Die Erfindung wird im folgenden anhand von bevorzugten Ausführungsbeispielen näher erläutert.

Die in den Ausführungsbeispielen zur Herstellung der Fettketone eingesetzten technischen, ungesättigten Fettsäuren wiesen die folgenden C-Kettenverteilungen (in %) sowie Jodzahlen auf:

| Fettsäure | $C_{14}$ | $C_{16}$ | $C_{18}$ | $C_{20}$ | Jodzahl |
|---|---|---|---|---|---|
| Ölsäure | 1 | 6 | 92 | 1 | 89 |
| Elaidinsäure | 1 | 6 | 92 | 1 | 89 |
| Petroselinsäure | 0 | 3 | 96 | 1 | 91 |
| Chaulmoograsäure | 0 | 3 | 85 | 12 | 93. |

Die Bestimmung der Klettfarbzahlen erfolgte nach 30 min Bleichung mit 5 Gew.-% $H_2O_2$-Lösung (35 %-ig). Die Messung erfolgte bei einer Konzentration von 5 Gew.-% Aniontensid, pH = 7 sowie unter Verwendung einer 1 cm-Küvette und eines Blaufilters (400 - 465 nm).

Beispiel 1.

Bis(heptadecenyl)keton-sulfonat.

A. Bis(heptadecenyl)keton (aus technischer Ölsäure).

In einen 4 l-Dreihalskolben mit Rührer, Innenthermometer, Kühler und Dosiertrichter wurden 57,0 g (0,2 mol) technische Ölsäure vorgelegt und mit 40,4 g (1,0 mol) Magnesiumoxid vermischt. Das Reaktionsgemisch wurde zunächst 30 min unter intensivem Rühren auf 340 °C aufgeheizt und anschließend innerhalb von ca. 45 min mit weiteren 225,6 g (0,8 mol) technischer Ölsäure versetzt. Nach einer Reaktionszeit von insgesamt 240 min wurde auf 100 °C abgekühlt; der Katalysator wurde über eine dampfbeheizte Glasfilternutsche abgetrennt.

Die Titelverbindung wurde als hellgelbe Flüssigkeit mit einer Ausbeute von ca. 450 g (90 % d. Th.) erhalten.

| | |
|---|---|
| Jodzahl | 108 |
| CO-Zahl | 55 |
| mittleres Molgewicht | 468. |

B. Bis(heptadecenyl)keton-sulfonat.

468 g (1 mol) Bis(heptadecenyl)keton aus der Stufe A wurden vorgelegt und bei 40 bis 45 °C mit 96 g (1,2 mol) gasförmigem Schwefeltrioxid entsprechend einem Molverhältnis von Schwefeltrioxid zu olefinischen Doppelbindungen von 1 : 0,6 umgesetzt.

Das Schwefeltrioxid wurde durch Erhitzen aus einer entsprechenden Menge 65 %-igem Oleum ausgetrieben, mit Stickstoff auf eine Konzentration von 5 Vol.-% verdünnt und innerhalb von 35 min in das Keton eingeleitet, wobei die Temperatur des Reaktionsgemisches durch Kühlen stets unter 60 °C gehalten wurde.

Nach der Sulfonierung wurde das saure Reaktionsgemisch in eine Lösung von 52 g (1,3 mol) Natriumhydroxid in 250 ml Wasser eingerührt, 4 Stunden bei 95 °C hydrolysiert und anschließend mit Mineralsäure auf einen pH von 7 eingestellt.

Kenndaten des Produktes:

| | |
|---|---|
| Aniontensid (DGF-H-III-10) | 9,6 % = 0,0168 mval/g |
| Unsulfierte Anteile (DGF-G-III-6b) | 6,2 % |
| Sulfat (berechnet als $Na_2SO_4$) | 1,5 % |
| Wasser (nach Fischer) | 82,7 % |
| Gesamtschwefelgehalt | 0,87 % |
| Klettfarbzahl | 56 |
| Mittleres Molgewicht | 570 |

C. Bis(heptadecenyl)keton-sulfonat.

Bei der Wiederholung der oben genannten Sulfonierung mit 162 g (2,0 mol) Schwefeltrioxid entsprechend einem Molverhältnis von Schwefeltrioxid zu olefinischen Doppelbindungen von 1 : 1 wurde das Schwefeltrioxid innerhalb von 65 min in die Reaktionsmischung eingeleitet; Nach Beendigung der Sulfonierung wurde das Rohprodukt mit 98 g (2,2 mol) Natriumhydroxid in 500 ml Wasser unter den im übrigen gleichen Bedingungen hydrolysiert und neutralisiert.

| Kenndaten des Produktes: | |
|---|---|
| Aniontensid (DGF-H-III-10) | 17,3 % = 0,0257 mval/g |
| Unsulfierte Anteile (DGF-G-III-6b) | 3,5 % |
| Sulfat (berechnet als $Na_2SO_4$) | 1,2 % |
| Wasser (nach Fischer) | 78,0 % |
| Gesamtschwefelgehalt | 1,09 % |
| Klettfarbzahl | 88 |
| Mittleres Molgewicht | 672 |

Beispiel 2.

Bis(heptadecenyl)keton-sulfonat.

A. Bis(heptadecenyl)keton (aus technischer Elaidinsäure).

Analog zu der in Beispiel 1, Stufe A, beschriebenen Arbeitsweise wurde aus 282 g technischer Elaidinsäure 440 g (89 % d. Th.) Bis(heptadecenyl)keton erhalten.

| | |
|---|---|
| Jodzahl | 110 |
| CO-Zahl | 54 |
| mittleres Molekulargewicht | 461. |

B. Bis(heptadecenyl)keton-sulfonat.

Analog zu der in Beispiel 1, Stufe C, beschriebenen Arbeitsweise wurden 463 g (1,0 mol) des vorstehend erhaltenen Bis(heptadecenyl)ketons sulfoniert.

| Kenndaten des Produktes: | |
|---|---|
| Aniontensid (DGF-H-III-10) | 16,5 % = 0,0248 mval/g |
| Unsulfierte Anteile (DGF-G-III-6b) | 3,9 % |
| Sulfat (berechnet als $Na_2SO_4$) | 1,2 % |
| Wasser (nach Fischer) | 78,4 % |
| Gesamtschwefelgehalt | 1,06 % |
| Klettfarbzahl | 83 |
| Mittleres Molgewicht | 665 |

Beispiel 3.

Bis(heptadecenyl)keton-sulfonat.

A. Bis(heptadecenyl)keton (aus technischer Petroselinsäure).

Analog zu der in Beispiel 1, Stufe A, beschriebenen Arbeitsweise wurde aus 282 g technischer Petroselinsäure 450 g (90 % d. Th.) Bis(heptadecenyl)keton erhalten.

| | |
|---|---|
| Jodzahl | 110 |
| CO-Zahl | 55 |
| mittleres Molekulargewicht | 461. |

B. Bis(heptadecenyl)keton-sulfonat.

Analog zu der in Beispiel 1, Stufe C, beschriebenen Arbeitsweise wurden 463 g (1,0 mol) des vorstehend erhaltenen Bis(heptadecenyl)ketons sulfoniert.

| Kenndaten des Produktes: | |
|---|---|
| Aniontensid (DGF-H-III-10) | 17,1 % = 0,0257 mval/g |
| Unsulfierte Anteile (DGF-G-III-6b) | 3,6 % |
| Sulfat (berechnet als $Na_2SO_4$) | 1,3 % |
| Wasser (nach Fischer) | 78,0 % |
| Gesamtschwefelgehalt | 1,12 % |
| Klettfarbzahl | 91 |
| Mittleres Molgewicht | 665 |

Beispiel 4.

Bis(1-dodecyl-cyclopenten-2)keton-sulfonat.

A. Bis(1-dodecyl-cyclopenten-2)keton (aus Chaulmoograsäure).

In der in Beispiel 1, Stufe A, beschriebenen Arbeitsweise wurden aus 280 g technischer Chaulmoograsäure (2-Cyclopenten-1-tridecansäure) 440 g (89 % d. Th.) Bis(1-dodecyl-cyclopenten-2)keton als hellgelbe Flüssigkeit erhalten.

| | |
|---|---|
| Jodzahl | 112 |
| CO-Zahl | 56 |
| mittleres Molekulargewicht | 453. |

B. Bis(1-dodecyl-cyclopenten-2)keton-sulfonat.

Analog zu der in Beispiel 1, Stufe C, beschriebenen Arbeitsweise wurden 453 g (1,0 mol) Bis(1-dodecyl-cyclopenten-2)keton sulfoniert.

| Kenndaten des Produktes: | |
|---|---|
| Aniontensid (DGF-H-III-10) | 16,0 % = 0,0257 mval/g |
| Unsulfierte Anteile (DGF-G-III-6b) | 4,3 % |
| Sulfat (berechnet als $Na_2SO_4$) | 1,6 % |
| Wasser (nach Fischer) | 78.1 % |
| Gesamtschwefelgehalt | 1,18 % |
| Klettfarbzahl | 105 |
| Mittleres Molgewicht | 657 |

## Ansprüche

1. Sulfonierte Fettketone, erhältlich durch Sulfonierung von Fettketonen der allgemeinen Formel I
$R^1$-CO-$R^2$ (I)
in der
$R^1$ einen Kohlenwasserstoffrest mit 15 bis 21 Kohlenstoffatomen und einer olefinischen Doppelbindung oder mehreren und
$R^2$ einen gegebenenfalls eine olefinische Doppelbindung oder mehrere aufweisenden Kohlenwasserstoffrest mit 1 bis 21 Kohlenstoffatomen
bedeuten,
sowie anschließender Neutralisation und Hydrolyse bzw. Alkoholyse der Sulfonierungsprodukte.

2. Sulfonierte Fettketone nach Anspruch 1, erhältlich durch Sulfonierung von Fettketonen der allgemeinen Formel I, in der
$R^1$ und $R^2$ jeweils gleiche Kohlenwasserstoffreste mit 15 bis 21 Kohlenstoffatomen und einer olefinischen Doppelbindung oder mehreren bedeuten.

3. Sulfonierte Fettketone nach Anspruch 1 oder 2, erhältlich durch Sulfonierung von Fettketonen der allgemeinen Formel I, in der $R^1$ und $R^2$ jeweils gleiche Kohlenwasserstoffreste aus der von Pentadecylen, Heptadecylen, 1-Dodecyl-cyclopenten-2, Nonadecylen und Heneicosylen gebildeten Gruppe bedeuten.

4. Sulfonierte Fettketone nach mindestens einem der Ansprüche 1 bis 3, dadurch gekennzeichnet, daß die Sulfonierung mit einem mit einem inerten Trägergas verdünnten Schwefeltrioxidstrom erfolgt.

5. Sulfonierte Fettketone nach mindestens einem der Ansprüche 1 bis 4, dadurch gekennzeichnet, daß die Sulfonierung in Abwesenheit von Lösemitteln erfolgt.

6. Sulfonierte Fettketone nach mindestens einem der Ansprüche 1 bis 5, dadurch gekennzeichnet, daß die Sulfonierung in einem Verhältnis von 0,5 bis 1,8, insbesondere 0,6 bis 1,5 und bevorzugt von 1,0 bis 1,3 Mol Schwefeltrioxid pro Mol der in den Fettketonen der allgemeinen Formel I enthaltenen olefinischen Doppelbindungen durchgeführt wird.

7. Sulfonierte Fettketone nach mindestens einem der Ansprüche 1 bis 6, dadurch gekennzeichnet, daß die Sulfonierung bei Temperaturen von 15 bis 80, insbesondere 40 bis 70 °C erfolgt.

8. Sulfonierte Fettketone nach mindestens einem der Ansprüche 1 bis 7, dadurch gekennzeichnet, daß die Neutralisation und Hydrolyse bzw. Alkoholyse mit wässrigen und/oder alkoholischen Lösungen bzw. Suspensionen aus der von Alkalimetallhydroxiden bzw. -$C_1$-$C_4$-alkoxiden, Erdalkalimetalloxiden bzw. -hydroxiden, Ammoniak, mono-, Di- bzw. Tri-$C_1$-$C_4$-alkylaminen und Mono-, Di- bzw. Tri-hydroxy-$C_2$-$C_4$-alkylaminen gebildeten Gruppe erfolgt.

9. Sulfonierte Fettketone nach mindestens einem der Ansprüche 1 bis 8, dadurch gekennzeichnet, daß die Neutralisation und Hydrolyse bzw. Alkoholyse bei pH-Werten im Bereich von 6 bis 14, insbesondere 8 bis 12, erfolgt.

10. Sulfonierte Fettketone nach mindestens einem der Ansprüche 1 bis 9, dadurch gekennzeichnet, daß die Neutralisation und Hydrolyse bzw. Alkoholyse bei Temperaturen von 70 °C innerhalb von 15 bis 240 min erfolgt.

11. Verfahren zur Herstellung von sulfonierten Fettketonen, gekennzeichnet durch eine Sulfonierung von Fettketonen der allgemeinen Formel I
$R^1$-CO-$R^2$ (I)
in der
$R^1$ einen Kohlenwasserstoffrest mit 15 bis 21 Kohlenstoffatomen und einer olefinischen Doppelbindung oder mehreren und

$R^2$ einen gegebenenfalls eine olefinische Doppelbindung oder mehrere aufweisenden Kohlenwasserstoffrest mit 1 bis 21 Kohlenstoffatomen
bedeuten,
sowie anschließender Neutralisation und Hydrolyse bzw. Alkoholyse der Sulfonierungsprodukte.

12. Verfahren nach Anspruch 11, gekennzeichnet durch eine Sulfonierung von Fettketonen der allgemeinen Formel I, in der $R^1$ und $R^2$ jeweils gleiche Kohlenwasserstoffreste mit 15 bis 21 Kohlenstoffatomen und einer olefinischen Doppelbindung oder mehreren bedeuten.

13. Verfahren nach Anspruch 11 oder 12, gekennzeichnet durch eine Sulfonierung von Fettketonen der allgemeinen Formel I, in der $R^1$ und $R^2$ jeweils gleiche Kohlenwasserstoffreste aus der von Pentadecylen, Heptadecylen, 1-Dodecyl-cyclopenten-2, Nonadecylen und Heneicosylen gebildeten Gruppe bedeuten.

14. Verfahren nach mindestens einem der Ansprüche 11 bis 13, dadurch gekennzeichnet, daß die Sulfonierung mit einem mit einem inerten Trägergas verdünnten Schwefeltrioxidstrom erfolgt.

15. Verfahren nach mindestens einem der Ansprüche 11 bis 14, dadurch gekennzeichnet, daß die Sulfonierung in Abwesenheit von Lösemitteln erfolgt.

16. Verfahren nach mindestens einem der Ansprüche 11 bis 13, dadurch gekennzeichnet, daß die Sulfonierung in einem Verhältnis von 0,5 bis 1,8, insbesondere 0,6 bis 0,6 bis 1,5 und bevorzugt von 1,0 bis 1,3 Mol Schwefeltrioxid pro Mol der in den Fettketonen der allgemeinen Formel I enthaltenen olefinischen Doppelbindungen durchgeführt wird.

17. Verfahren nach mindestens einem der Ansprüche 11 bis 16, dadurch gekennzeichnet, daß die Sulfonierung bei Temperaturen von 15 bis 80, insbesondere 40 bis 70 °C erfolgt.

18. Verfahren nach mindestens einem der Ansprüche 11 bis 17, dadurch gekennzeichnet, daß die Neutralisation und Hydrolyse bzw. Alkoholyse mit wässrigen und/oder alkoholischen Lösungen bzw. Suspensionen aus der von Alkalimetallhydroxiden bzw. -$C_1$-$C_4$-alkoxiden, Erdalkalimetalloxiden bzw. -hydroxiden, Ammoniak, mono-, Di- bzw. Tri-$C_1$-$C_4$-alkylaminen und Mono-, Di- bzw. Tri-hydroxy-$C_2$-$C_4$-alkylaminen gebildeten Gruppe erfolgt.

19. Verfahren nach mindestens einem der Ansprüche 11 bis 18, dadurch gekennzeichnet, daß die Neutralisation und Hydrolyse bzw. Alkoholyse bei pH-Werten im Bereich von 6 bis 14, insbesondere 8 bis 12, erfolgt.

20. Verfahren nach mindestens einem der Ansprüche 11 bis 19, dadurch gekennzeichnet, daß die Neutralisation und Hydrolyse bzw. Alkoholyse bei Temperaturen von mindestens 70 °C innerhalb von 15 bis 240 min erfolgt.

21. Oberflächenaktive Mittel, enthaltend einzeln oder in Mischung sulfonierte Fettketone der allgemeinen Formel II

$R^3$-CO-$R^4$ (II)

in der
$R^3$ eine Gruppe der Formel IIIa bzw. IIIb

$CH_3$-$(CH_2)_x$-CH($OR^5$)-$(CH_2)_y$-CH($SO_3M$)-$(CH_2)_z$-$CH_2$- (IIIa)

$CH_3$-$(CH_2)_x$-CH($SO_3M$)-$(CH_2)_y$-CH($OR^5$)-$(CH_2)_z$-$CH_2$- (IIIb)

bedeutet,
in denen
$R^5$ Wasserstoff oder Alkyl mit 1 bis 3 Kohlenstoffatomen,
x und z Zahlen im Bereich von 0 bis 17,
y die Zahlen 0,1 oder 2 und
M ein Alkalimetall- oder ein Äquivalent eines Erdalkalimetallions oder ein gegebenenfalls mono-, di- oder tri-$C_1$-$C_4$-alkyl- bzw. mono-, di- oder tri-$C_2$-$C_4$-hydroxyalkylsubstituiertes Ammoniumion ist,
wobei die Summe von x + y + z eine Zahl im Bereich von 13 bis 17 ergibt,
oder $R^3$ einen Rest, der durch Abspaltung eines Moleküls Wasser aus den Gruppen IIIa bzw. IIIb gebildet ist,
und
$R^4$ wie $R^3$ definiert ist oder einen gegebenenfalls eine olefinische Doppelbindung oder mehrere aufweisenden Kohlenwasserstoffrest mit 1 bis 21 Kohlenstoffatomen
bedeutet.

22. Oberflächenaktive Mittel, enthaltend einzeln oder in Mischung sulfonierte Fettketone der allgemeinen Formel IV

$R^6$-$(CH_2)_{12}$-CO-$(CH_2)_{12}$-$R^7$ (IV)

in der
$R^6$ eine Gruppe der Formel V

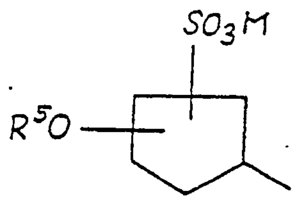

(V),

wobei $R^5$ und M wie im Anspruch 21 definiert sind,
oder $R^6$ einen Rest bedeutet, der durch Abspaltung eines Moleküls Wasser aus der Gruppe der Formel V gebildet ist,
und
$R^7$ einen Cyclopent-(2)-yl-Rest bedeutet oder wie $R^6$ definiert ist.

23. Verwendung der sulfonierten Fettketone nach mindestens einem der Ansprüche 1 bis 10 als oberflächenaktive Mittel.

Europäisches Patentamt

# EUROPÄISCHER RECHERCHENBERICHT

Nummer der Anmeldung

EP 89 11 5964

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-2 037 974 (F. GUENTHER et al.)<br>--- | | C 07 C 309/07<br>C 07 C 303/02<br>C 11 D 1/12 |
| A | US-A-2 308 841 (J.H. WERNTZ)<br>----- | | |
| | | | **RECHERCHIERTE SACHGEBIETE (Int. Cl.5)** |
| | | | C 07 C 309/00 |

Der vorliegende Recherchenbericht wurde für alle Patentansprüche erstellt

| Recherchenort | Abschlußdatum der Recherche | Prüfer |
|---|---|---|
| DEN HAAG | 07-11-1989 | VAN GEYT J.J.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)